# EUROPEAN PATENT SPECIFICATION

(11) **EP 3 592 223 B1**
(45) Date of publication and mention of the grant of the patent: **25.11.2020**
(21) Application number: 18708701.0
(22) Date of filing: 08.03.2018
(51) Int. Cl.: A61B 5/0428, H01B 11/00, A61B 5/0408

(54) **ECG CABLE FOR CONNECTION WITH AN ECG MONITOR**
EKG-KABEL ZUR VERBINDUNG MIT EINEM EKG-MONITOR
CÂBLE ECG POUR CONNEXION À UN MONITEUR ECG

(30) Priority: 08.03.2017 EP 17159884
(43) Date of publication of application: 15.01.2020
(73) Proprietor: Koninklijke Philips N.V., 5656 AG Eindhoven (NL)
(72) Inventor: VERBAKEL, Frank, 5656 AE Eindhoven (NL); LAMBERT, Nicolaas, 5656 AE Eindhoven (NL); KAHLMAN, Josephus, Arnoldus, Henricus, Maria, 5656 AE Eindhoven (NL); WOERLEE, Pierre, Hermanus, 5656 AE Eindhoven (NL); DE SAMBER, Marc, Andre, 5656 AE Eindhoven (NL)
(74) Representative: Philips Intellectual Property & Standards
(86) International application number: PCT/EP2018/055702
(87) International publication number: WO 2018/162616

(56) References cited:
- CN-U- 201 918 188
- CN-U- 203 953 644
- US-A- 5 491 299
- US-A1- 2014 025 154
- NI CR ET AL: "Alloy Data Table - Resistance Heating Wire Iron-Chrome-Aluminum (Fe-Cr-Al) Alloy -KA1 Alloy Data", ADT2001.12.17.KA1.MET 3508 INDEPENDENCE DRIVE FORT WAYNE, IN 46808 TOLL FREE: 888.496.3626 APPROX. MELTING POINT 1500&#XF0B0;C MAX. CONTINUOUS OPERATING TEMP 294.0816 2.3048 0.3486 11.7926 29.5646 72.4084 207.6856 2.0525 0.4396 18.7510 23.4458 64.481, vol. 8804009730544164309858187249622555780467, no. 1, 22 April 2014 (2014-04-22), pages 375966-16, XP055403932,

## Description

### FIELD OF THE INVENTION

The present invention relates to an ECG cable for connection with an ECG monitor.

### BACKGROUND OF THE INVENTION

There is a strong trend towards wearable devices in healthcare and/or lifestyle. This is related among others to increasing demand for ambulatory monitoring ease-of-use, patient comfort, workflow management, etc. One of the key characteristics and requirements of such wearable device is its specific form factor, for which ECG is the most challenging to meet. For instance, up to 10 electrodes and wires (often called "leads" or "lead wires") can be used to generate a so-called 12 lead ECG representation on the monitor.

A trunk cable is used as an interface between lead sets comprising 3, 4, 5 or 6 electrodes and the ECG monitor. To protect the monitoring equipment and to ensure that in case of a defibrillation pulse minimally 90 % of the energy is directed to the patient (as needed to reset the heart) serial protection resistors capable of high-energy absorption are required for every wire. Said resistors (typically 1 kOhm for respiration and 3.3 kOhm for ECG-related lead wires) are located in the trunk cable connector and are able to absorb minimally approx. 15 J (for 1 kOhm) each, which requires a rather bulky trunk cable connector.

Standard ICU ECG patient lead sets should have low inductance to allow bio-impedance respiration measurements at 48 kHz.

For application in the operating room (OR) dedicated OR ECG patient lead sets comprise additional 6.8 mH inductors and low-power 9 kOhm resistors in series to suppress RF signals from the use of cauterization devices and electrical knifes. Due to the high (10 kOhm) resistance the bio-impedance (respiration) measurement is not possible, which is acceptable for the OR-application as ventilation and capnography signals are available as an alternative. When the ECG monitor detects the 10 kOhm (9 + 1 kOhm) serial resistance, bio-impedance is automatically disabled.

Conventional ECG cables for connection with an ECG monitor are e.g. disclosed in CN 203953644 U and CN 201918188 U.

Development currently aims at the combination of small sized wearable measurement modules (as an embodiment of ECG monitor, as understood herein), non-galvanic (inductive) powering and wireless radio communication as an enabler for advanced and high quality wearable patient monitoring. However, currently the dimensions of an ECG trunk cable connector (e.g. approx. 8 x 4 x 1.8 cm) are too large to be compatible with the targeted small-sized measurement modules.

### SUMMARY OF THE INVENTION

It is an object of the present invention to provide an ECG cable for connection with an ECG monitor that is more compatible with the targeted small-sized measurement modules.

According to the present invention an ECG cable for connection with an ECG monitor is presented comprising
- a core,
- a resistive wire cable comprising a plurality of resistive wires wound around the core and isolated with respect to each other, wherein each resistive wire has a resistance of at least 500 Ω and wherein the resistive wires are made of a material having a resistivity of at least 0.135 µΩ*m, and
- two or more flexible lead wires, each connected to a respective resistive wire at its one end and to an electrode at its other end.

Preferred embodiments of the invention are defined in the dependent claims.

The present invention is based on the idea to replace the conventionally used trunk cable and the conventionally used bulky trunk cable connector by a new cable, which comprises a (thin) trunk cable (formed by the core and the resistive wire cable) and (at least partly) flexible lead wires to the electrodes. In particular, it is proposed to create a miniaturized ECG cable-set having distributed impedances (resistors, inductors) in the lead set and/or trunk cable based on a winding technology preferably using resistive wires and optionally high-permeability (e.g. ferrite) material. This allows removing the need for a separate large trunk cable connector (currently the normal holder of the resistors), and therefore strongly reduces the size of the cable set. Thereby, workflow and patient friendliness is highly improved. A wide range of wire diameters and winding geometries is possible enabling both low and high inductance cables.

Thus, according to the present invention a multi-resistive wire cable is used to form a wire-wound resistor. Such a resistive wire cable may also be called a multi-core cable due to the use of multiple resistive wires (sometimes also called "cores"; generally also known as "resistance wires"). That a lead wire is connected with a respective resistive wire in this context means that the lead wire is either directly connected to the respective resistive wire or via a connector.

In a preferred embodiment the resistive wire cable is spirally wound around at least part of the core. This enables to design the desired resistivity of the resistive wire cable in an easy manner.

As an advantageous material the resistive wires of the wire cable is substantially made of a FeCrAl alloy, such as a material distributed under the trademark Kanthal, which allows achieving the desired resistivity.

In a preferred implementation the resistive wires have a diameter in the range of 50 to 500 µm, in particular in the range of 100 to 200 µm, which ensures the desired miniaturization flexibility.

There are various embodiments for implementing the resistive wires. In a preferred embodiment a resistive wire comprises a bundle of multiple wires. This increases the flexibility and reduces the chance of repeated stress failures.

In an embodiment, the resistive wire cable may comprise two bifilar wound wires for conducting opposite current flows. This provides for a reduced self-inductance.

In still another embodiment the resistive wire cable may comprise parallel resistive wires wound in opposite direction around the core, which also achieves the desired flexibility and miniaturization.

The resistive wire cable may also comprise a woven shield of non-isolated wires. This will also strongly reduce the self-inductance.

According to another embodiment the resistive wire cable comprises a plurality of resistive wires, each connected to a lead wire, wherein said plurality of resistive wires are wound in parallel around the core. The advantage is that two or more resistors can be provided in the same wire with only a small increase in length.

The core of the ECG may be made of plastic, ferrite or a material having a high permeability, in particular having a relative permeability above 10, preferably above 100. This further increases the inductivity, if desired.

In another embodiment the pitch of the windings of the resistive wire cable varies over the length of the resistive wire. In this way regions of higher and lower resistance can be created and cables of different lengths can be easily obtained.

Preferably, each resistive wire has a resistance of at least 1 kΩ. The diameter, length and material are chosen accordingly to achieve the desired value of the resistance.

In an embodiment the resistive wires are made of a material having a resistivity in the range of 1.35 to 1.45 µΩ*m.

### BRIEF DESCRIPTION OF THE DRAWINGS

These and other aspects of the invention will be apparent from and elucidated with reference to the embodiment(s) described hereinafter. In the following drawings
Fig. 1 shows a schematic drawing of the general layout of an ECG cable according to the present invention,
Fig. 2 shows a schematic drawing of an embodiment of an ECG cable according to the present invention with multiple parallel wires,
Fig. 3 shows a schematic drawing of an embodiment of an ECG cable according to the present invention spirally wound wires in parallel,
Fig. 4 shows a schematic drawing of an embodiment of an ECG cable according to the present invention with spirally wound wires in antiparallel direction,
Fig. 5 shows a schematic drawing of an embodiment of an ECG cable according to the present invention with woven wires,
Figs. 6A and 6B show schematic drawings of two embodiments of an ECG cable according to the present invention with spirally wound wires with different pitches,
Figs. 7A and 7B show schematic drawings of two embodiments of an ECG cable according to the present invention with different cores, and
Fig. 8 shows a schematic drawing of an embodiment of an ECG cable according to the present invention with a spirally wound wire having a variable pitch.

### DETAILED DESCRIPTION OF EMBODIMENTS

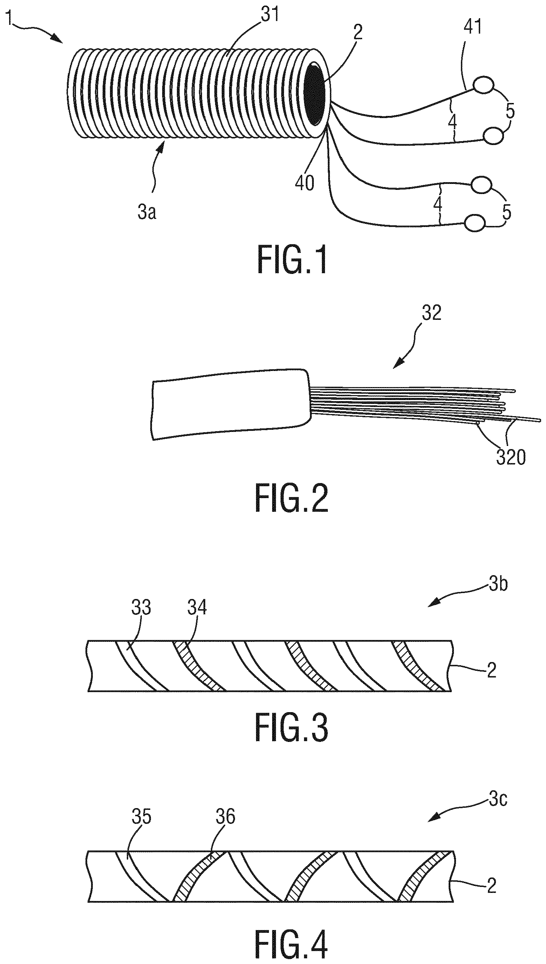

Fig. 1 shows a schematic drawing of the general layout of an ECG cable 1 according to the present invention. It comprises a core 2, a resistive wire cable 3a wound, e.g. like a helix, around the core and formed by resistive wires 31, and two or more flexible lead wires 4, each connected to a corresponding resistive wire 31 at its one end 40 and to an electrode 5 at its other end 41. There are various embodiments of arranging the resistive wire cable and of forming the resistive wire cable by resistive wires, as will be illustrated and explained in the following.

There a number of potentially useful metal materials that can meet all or part of the requirements of the proposed solution, e.g. on resistive values, Temperature Coefficient of Resistance (TCR), mechanical properties, etc. A preferred material is FeCrAl. A family of iron-chromium-aluminum (FeCrAl) alloys used in a wide range of resistance and high-temperature applications are e.g. available under the trade name Kanthal. Kanthal FeCrAl alloys consist of mainly iron, chromium (20-30 %) and aluminum (4-7.5 %), e.g. Kanthal A-1, Kanthal D, Kanthal AF, have a resistivity of 1.35-1.45 µΩ*m. This material, for instance, allows designing a resistor with the desired resistance of 1 kOhm by winding this wire around a plastic core. However, several other stable resistive metal alloys exist with similar or lower resistivity, which may alternatively be applied.

Taking a temperature increase of 50 K (due to the energy of the defibrillation pulse) as a suggested specification, a required length of approx. 13 m of 0.16 mm diameter resistive wire may be used to reach 1 kOhm. Embedding a 13 m long resistive wire in the envisioned 0.5 m length of a lead wire requires a helix pitch of 0.25 mm. If a 4 times higher temperature increase (200 K) would be allowed the ECG wire length could be reduced to 6.5 m using 0.11 mm diameter resistive wire. For a resistance of 3.3 kOhm the diameter can be reduced by a factor of sqrt (3.3) = 1.8 at the same length (because the absorbed energy due to defibrillation pulse in the higher resistance is reduced, the thermal mass of the wire is allowed to be lower). This would allow either using a non-winded solution or a much more relaxed winding technology.

Calculated values are illustrated in the following table (for the assumption that there is a resistive heat pulse for defibrillation with 40 J; generally the defibrillation pulse is approx. 360 J, but at 1 kOhm only approx. 40 J ends up in the lead wires):

| | | Temperature increase | |
|---|---|---|---|
| | | 50 K | 200 K |
| Min. wire volume | cm³ | 0.245 | 0.061 |
| Min. wire length | m | 13.0 | 6.5 |
| Min. wire diameter | mm | 0.155 | 0.110 |
| Helix winding diameter | mm | 1.845 | 1.890 |
| Helix inner diameter Number of turns | mm | 1.690 2242 | 1.781 1094 |
| Helix pitch | mm | 0.223 | 0.457 |
| Max wire insulation thickness | mm | 0.034 | 0.174 |
| Self-inductance | µH | 33.8 | 8.4 |
| Reactance at f_carrier | Ohm | 10.2 | 2.5 |

Respiration measurements require low inductive lead wires. The self-inductance of a 13 m long resistive wire wound to a helix of 0.5 m long is 32 µH. In many ECG applications also respiration is measured by using bio-impedance at a carrier frequency of typically 48 kHz. The reactance of the helix at 48 kHz is approx. 10 Ohm, and about 2.5 Ohm for a 6.5 m long wire.

The ECG cable may be fabricated by winding the resistive wire cable on a metal rod, where after the rod is removed and isolation is applied at the outside of the helix. Finally the inside may be filled with a silicone rubber with high flexibility. In a possible and suggested future reel-to-reel production method the helix could be wound continuously around plastic core and immediately covered with insulation via an extrusion process.

There are a number of embodiments for implementing the proposed idea, which shall be further elucidated in the following.

Fig. 2 shows a schematic drawing of an embodiment of a resistive wire 32 according to the present invention with multiple parallel wires 320. The lead wires are not shown in this figure. Similar to electricity wires a single resistive wire 31 as shown in Fig. 1 can be replaced by a wire 32 comprising multiple thin wires 320. This will increase the flexibility and reduce the chance of repeated stress failures. The total volume of resistive wire 32 should be kept constant to ensure that the thermal energy still can be absorbed. The total diameter of the wire 32 will be slightly increased due to the small air gap between the thin wires 320. A single wire with a diameter of 150µm can thus be replaced by e.g. 36 wires of 25µm or 9 wires of 50 µm diameter.

Fig. 3 shows a schematic drawing of an embodiment of an ECG cable according to the present invention spirally wound wires 33, 34 forming the resistive wire cable 3b. The lead wires are not shown in this figure. Bio-impedance measurements (e.g. for respiration measurement) require a low self-inductance lead set, which requires reduction of the magnetic stray field as much as possible. This can be achieved by bifilar wound (resistive) wires 33, 34, where the opposite current flows in the adjacent wire 33, 34. In this case an additional (normal) wire may be used as with this winding the beginning and end are the same location. Alternatively, a normal winding may be added to end on the opposite side of the lead wire.

An alternative embodiment is depicted in Fig. 4 showing a schematic drawing of an embodiment of an ECG cable according to the present invention with spirally wound wires in antiparallel direction. This resistive wire cable 3c uses parallel (resistive) wires 35, 36 wound in helixes having an opposite rotational direction (like the double helix structure in a DNA molecule).

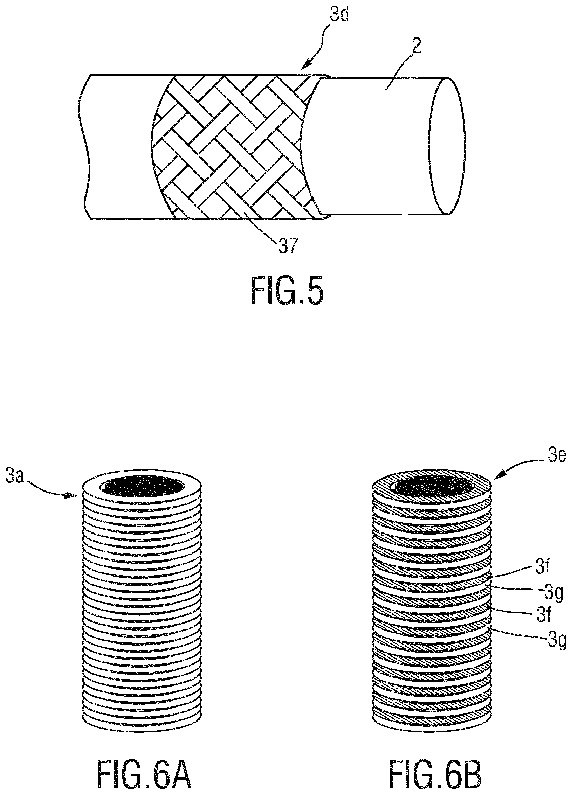

Fig. 5 shows a schematic drawing of an embodiment of an ECG cable according to the present invention with woven wires. In this embodiment, the resistive wire cable 3d is formed by a woven shield of non-isolated wires 37. Again, instead of a single wire (e.g. with a pitch of 0.225 µm) of 150 µm diameter, a coax woven structure of e.g. 10 wires of 50 µm diameter with the same pitch can be used. When an even number of wires is used combined with this structure, the self-inductance of the single wires will cancel, reducing the self-inductance to a very low level. This will reduce the noise and make respiration detection possible. In an embodiment the braiding angle should be as in the helix wound wire, as e.g. shown in Fig. 1, i.e. in practice a very different angle is used than the angle shown in Fig. 5.

Another disturbing and hence to-be-solved effect is the tribo-electric effect or handling-noise that causes impact-related "slapping" noise as the cable hits a mechanical stiffness or is stepped upon during use. This is related to capacitance, specifically the change in capacitance that takes place as the insulation or dielectric is deformed. The best work-around is by using soft, impact-absorbing insulation and jacket materials in a very solid construction with ample fillers to ensure that the cable retains its shape.

Figs. 6A and 6B show schematic drawings of two embodiments of an ECG cable according to the present invention with one spirally wound wire cable 3a (Fig. 6A) and a wire cable arrangement 3e with two spirally (parallel) wound wires cables 3f, 3g (Fig. 6B). Instead of having 5 or 10 separate lead wires with the wound wire resistors, the winding pitch can be increased to hold 5 or even 10 parallel wire resistors (i.e. wire cables) on a single core, e.g. with a 2 mm diameter (pitch 5 x 0.175 mm = approx. 0.9 mm) in approx. 2 m, or for 10 wires 5 m. If the diameter of the core is increased to approx. 8 mm, the pitch can again be reduced to 0.5 m for the 5 lead ECG cable. It should, however, be noted that these are only exemplary numbers for an exemplary implementation.

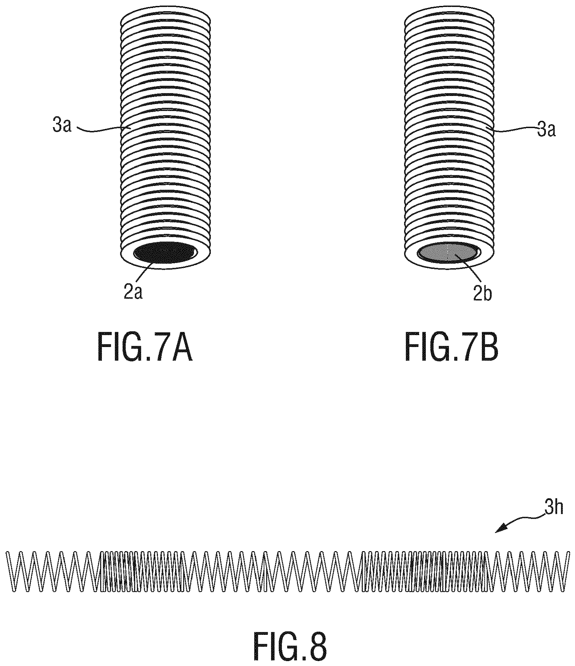

Figs. 7A and 7B show schematic drawings of two embodiments of an ECG cable according to the present invention with different cores. Instead of winding the resistive wire on an electro-magnetically inactive (e.g. plastic) core 2a, as shown in Fig. 7A, alternatively a high-µ (i.e. highly permeable, e.g. ferrite) core 2b can be used to increase the inductance, as shown in Fig. 7B. The inductance may be physically separated from the resistance by applying low resistive wire windings at a different position. This further miniaturizes the OR ECG cable set by omitting the need of separate 6.8 mH and 9 kOhm lead set connector.

This inductance can e.g. be implemented by replacing the magnetic inactive (e.g. plastic) core by a material with a µᵣ (relative permeability) of about 200 in a helix of 0.5 m length and 13 m wire. However, the ECG cable can also be redesigned or optimized to be functional with a lower resistance. Generally, the total resistance should remain above e.g. 1 kOhm, whereas the wire resistance can be changed and with this more or less length may be needed.

Fig. 8 shows a schematic drawing of an embodiment of an ECG cable according to the present invention with a spirally wound wire cable 3h having a variable pitch. By varying the winding pitch regions of higher resistance (for protection) and lower resistance can be created, while using the same resistive wire. In this way the same (standardized) cable technology can be applied to create different lengths of cables. Said regions may be indicated visually and used to cut the cable to the desired length. An additional advantage is that the length of low-impedance regions does not influence the total impedance too much so that different cable lengths may be feasible.

The present invention thus enables replacing the conventionally used trunk cable and trunk cable connector so that the ECG cable becomes more compact, lightweight and flexible.

While the invention has been illustrated and described in detail in the drawings and foregoing description, such illustration and description are to be considered illustrative or exemplary and not restrictive; the invention is not limited to the disclosed embodiments. The invention is defined by appended claims 1-13.

Any reference signs in the claims should not be construed as limiting the scope.

## Claims

1. ECG cable for connection with an ECG monitor, said ECG cable comprising:
- a core (2),
- a resistive wire cable (3a-3h) comprising a plurality of resistive wires (31-37) wound around the core and isolated with respect to each other, wherein each resistive wire has a resistance of at least 500 Ω and wherein the resistive wires are made of a material having a resistivity of at least 0.135 µΩ*m, and
- two or more flexible lead wires (4), each connected to a respective resistive wire at its one end (40) and to an electrode (5) at its other end (41).

2. ECG cable as claimed in claim 1,
wherein the resistive wire cable (3a-3h) is spirally wound around at least part of the core.

3. ECG cable as claimed in claim 1,
wherein the resistive wire cable (3a-3h) is substantially made of a FeCrAl alloy.

4. ECG cable as claimed in claim 1,
wherein the resistive wires (31-37) have a diameter in the range of 50 to 500µm, in particular in the range of 100 to 200µm.

5. ECG cable as claimed in claim 1,
wherein the resistive wire (32) comprises a bundle of multiple resistive wires (320).

6. ECG cable as claimed in claim 1,
wherein the resistive wire cable (3b) comprises two bifilar wound resistive wires (33, 34) for conducting opposite current flows.

7. ECG cable as claimed in claim 1,
wherein the resistive wire cable (3c) comprises parallel resistive wires (35, 36) wound in opposite direction around the core.

8. ECG cable as claimed in claim 1,
wherein the resistive wire cable (3d) comprises a woven shield of non-isolated wires (37).

9. ECG cable as claimed in claim 1,
wherein two or more resistive wire cable (3f, 3g) are wound in parallel around the core.

10. ECG cable as claimed in claim 1,
wherein the core (2a, 2b) is made of plastic, ferrite or a material having a high permeability, in particular having a permeability above 10, preferably above 100.

11. ECG cable as claimed in claim 2,
wherein the pitch of the windings of the resistive wire cable (3h) varies over the length of the resistive wire cable.

12. ECG cable as claimed in claim 1,
wherein each resistive wire has a resistance of at least 1 kΩ.

13. ECG cable as claimed in claim 1,
wherein the resistive wires (31-37) are made of a material having a resistivity in the range of 1.35 to 1.45 µΩ*m.

## Patentansprüche

1. EKG-Kabel für den Anschluss an einem EKG-Monitor, wobei das EKG-Kabel Folgendes umfasst:
- eine Ader (2),
- ein Widerstandsdrahtkabel (3a-3h) mit mehreren Widerstandsdrähten (31-37), die um die Ader gewickelt und voneinander isoliert sind, wobei die einzelnen Widerstandsdrähte einen Widerstand von mindestens 500 Ω aufweisen, und wobei die Widerstandsdrähte aus einem Material mit einem spezifischen Widerstand von mindestens 0,135 µΩ*m bestehen, und
- mindestens zwei flexible Zuleitungsdrähte (4), die jeweils an einem Ende (40) mit dem entsprechenden Widerstandsdraht und am anderen Ende (41) mit einer Elektrode (5) verbunden sind.

2. EKG-Kabel gemäß Anspruch 1,
wobei das Widerstandsdrahtkabel (3a-3h) spiralförmig um mindestens einen Teil der Adel gewickelt ist.

3. EKG-Kabel gemäß Anspruch 1,
wobei das Widerstandsdrahtkabel (3a-3h) im wesentlichen aus einer FeCrAl-Legierung besteht.

4. EKG-Kabel gemäß Anspruch 1,
wobei die Widerstandsdrähte (31-37) einen Durchmesser im Bereich von 50 bis 500 µm und insbesondere im Bereich von 100 bis 200 µm aufweisen.

5. EKG-Kabel gemäß Anspruch 1,
wobei der Widerstandsdraht (32) ein Bündel aus mehreren Widerstandsdrähten umfasst.

6. EKG-Kabel gemäß Anspruch 1,
wobei das Widerstandsdrahtkabel (3b) zwei zweiadrige gewickelte Widerstandsdrähte (33, 34) zum Leiten gegenläufiger Stromflüsse umfasst.

7. EKG-Kabel gemäß Anspruch 1,
wobei das Widerstandsdrahtkabel (3c) parallele Widerstandsdrähte (35, 36) aufweist, die in gegenläufiger Richtung um die Ader gewickelt sind.

8. EKG-Kabel gemäß Anspruch 1,
wobei das Widerstandsdrahtkabel (3d) eine gewebte Abschirmung aus nicht isolierten Drähten (37) umfasst.

9. EKG-Kabel gemäß Anspruch 1,
wobei mindestens zwei Widerstandsdrahtkabel (3f, 3g) parallel um die Ader gewickelt sind.

10. EKG-Kabel gemäß Anspruch 1,
wobei die Ader (2a, 2b) aus Kunststoff, Ferrit oder einem Material mit hoher Durchlässigkeit besteht, hierbei insbesondere einer Durchlässigkeit von mehr als 10 oder bevorzugt von über 100.

11. EKG-Kabel gemäß Anspruch 2,
wobei der Wickelschritt des Widerstandsdrahtkabels (3h) über die Länge des Widerstandsdrahtkabels variiert.

12. EKG-Kabel gemäß Anspruch 1,
wobei die einzelnen Widerstandsdrähte einen Widerstand von mindestens 1 kΩ aufweisen.

13. EKG-Kabel gemäß Anspruch 1,
wobei die Widerstandsdrähte (31-37) aus einem Material mit einem spezifischen Widerstand im Bereich von 1,35 bis 1,45 µΩ*m bestehen.

## Revendications

1. Câble ECG destiné au raccordement à un moniteur ECG, ledit câble ECG comprenant :
- une âme (2),
- un câble filaire résistif (3a-3h) comprenant une pluralité de fils résistifs (31-37) enroulés autour d'une âme et isolés les uns par rapport aux autres, dans lequel chaque fil résistif présente une résistance d'au moins 500 Ω et dans lequel les fils résistifs sont constitués d'une matière présentant une résistivité d'au moins 0,135 µΩ*m, et
- au moins deux fils conducteurs (4) flexibles, chacun étant raccordé à un fil résistif respectif à une extrémité (40) et à une électrode (5) à son autre extrémité (41).

2. Câble ECG selon la revendication 1,
dans lequel le câble filaire résistif (3a-3h) est enroulé en spirale autour d'au moins une partie de l'âme.

3. Câble ECG selon la revendication 1,
dans lequel le câble filaire résistif (3a-3h) est essentiellement constitué d'un alliage FeCrAl.

4. Câble ECG selon la revendication 1,
dans lequel les fils résistifs (31-37) présentent un diamètre dans la plage comprise entre 50 et 500 mm, en particulier dans la plage comprise entre 100 et 200 µm.

5. Câble ECG selon la revendication 1,
dans lequel le fil résistif (32) comprend un faisceau de plusieurs fils résistifs (320).

6. Câble ECG selon la revendication 1,
dans lequel le câble filaire résistif (3b) comprend deux fils résistifs (33, 34) enroulés bifilaires pour conduire des flux de courant opposés.

7. Câble ECG selon la revendication 1,
dans lequel le câble filaire résistif (3c) comprend des fils résistifs (35, 36) parallèles enroulés dans une direction opposée autour de l'âme.

8. Câble ECG selon la revendication 1, dans lequel le câble filaire résistif (3d) comprend un blindage tissé des files (37) non isolés.

9. Câble ECG selon la revendication 1,
dans lequel au moins deux câbles filaires résistifs (3f, 3g) sont enroulés en parallèle autour de l'âme.

10. Câble ECG selon la revendication 1,
dans lequel l'âme (2a, 2b) est en matière plastique, en ferrite ou en une matière présentant une perméabilité élevée, en particulier présentant une perméabilité supérieure à 10, de préférence supérieure à 100.

11. Câble ECG selon la revendication 2, dans lequel le pas des enroulements du câble filaire résistif (3h) varie sur la longueur du câble filaire résistif.

12. Câble ECG selon la revendication 1,
dans lequel chaque fil résistif présente une résistance d'au moins 1 kΩ.

13. Câble ECG selon la revendication 1,
dans lequel les fils résistifs (31-37) sont constitués d'une matière présentant une résistivité dans la plage comprise entre 1,35 et 1,45 µΩ*m.
